# EUROPEAN PATENT APPLICATION

(11) **EP 4 018 907 A1**
(43) Date of publication of application: **29.06.2022**
(21) Application number: 20853646.6
(22) Date of filing: 08.01.2020

(54) **DISPOSABLE SPIRAL ELECTRODE FOR FETAL SCALP**

(30) Priority: 20.08.2019 CN 201921354092 U
(71) Applicant: Qingdao Bright Medical Manufacturing Co., Ltd., Qingdao, Shandong 266107 (CN)
(72) Inventor: ZHAO, Dezheng, Qingdao, Shandong 266107 (CN); JING, Xiaoping, Qingdao, Shandong 266107 (CN)
(74) Representative: Cabinet Beau de Loménie
(86) International application number: PCT/CN2020/070796
(87) International publication number: WO 2021/031513

(57) **Abstract**

A disposable spiral electrode for a fetal scalp, comprising an electrode fixing column (6), a spiral electrode and an auxiliary electrode (6.1, 6.3) which are on the electrode fixing column (6), a twisted pair (4), an inner sleeve (8), an outer sleeve (1) and an outer sleeve fixing piece, wherein one end of the outer sleeve (1) is inserted into the outer sleeve fixing piece; the electrode fixing column (6) extends out of or retracts into the other end of the outer sleeve (1); one end of the twisted pair (4) sleeved with the inner sleeve (8) is respectively connected to the spiral electrode and the auxiliary electrode (6.1, 6.3), and the other end thereof is led out from within the outer sleeve fixing piece; an outer sleeve (2) in the outer sleeve fixing piece is movably sleeved on an inner sleeve (5) and may move relatively along the axial direction; one end of the outer sleeve (2) is in insertion connection with one end of the outer sleeve (1); a section of an inwardly concave arc (3.1) is arranged on a wire pressing ring (3) movably connected to one end of the inner sleeve (5); a groove (5.3) is formed in the edge of an end opening of the inner sleeve (5), and the wire pressing ring (3) is buckled on the end opening of the inner sleeve (5); and the inwardly concave arc (3.1) and the groove (5.3) form a meshing opening that clamps the twisted pair (4). The telescoping of the spiral electrode is convenient to control and accurate, and the twisted pair (4) is convenient to fix and reliable. The shape design of the electrode fixing column (6) is reasonable and safe to use.

## Description

### Technical field

The present invention belongs to the technical field of medical devices, and relates to a technique for clinical use in detecting a fetus to collect a fetal heartbeat signal, in particular, it is a disposable spiral electrode for fetal scalp.

### Background

Conventional detection of fetal heart rate and uterine contractions could be divided into external detection and internal detection, but the collected signal of the external detection may be unstable and intermittent as being affected by the surroundings, which may further cause misdiagnosis or the condition being delayed. The internal detection is more accurate than the external detection but it needs to fix a fetal scalp spiral electrode on the scalp of the fetus for collecting physiological parameters (such as producing ECG signals) during delivery when the membrane ruptures and the cervix opens up to 2 to 3 cm. A typical fetal scalp electrode mainly includes electrode fixing column, single spiral electrode, auxiliary electrode, twisted pair wire, outer protective sleeve and twisted pair fixing part which are assembled. The single spiral electrode and the auxiliary electrode are respectively arranged on the top and the side of the electrode fixing column and the twisted pair wire is inserted into the outer protective sleeve. One end of the outer protective sleeve is provided with the twisted pair fixing part and the electrode fixing column is extended or retracted from the other end of the outer protective sleeve. The twisted pair fixing part of the typical fetal scalp electrode is inconvenient to use and unreliable in fixing, and the shape of the electrode fixing column and the auxiliary electrode installation structure is also unreasonable.

### Technical Problem

How to design a disposable spiral electrode for fetal scalp with an electrode fixing column easy to operate to extend out or retract from and a reliable fixed twisted pair, further with a reasonable design of the shape of the electrode fixing column and safe to use, is a technical problem that needs to be solved urgently in the technical field.

### Summary

One aspect of the present invention is to provide a disposable spiral electrode for fetal scalp with an electrode fixing column easy to operate to extend out or retract from and a reliable fixed twisted pair, further with a reasonable design of the shape of the electrode fixing column and safe to use.

In order to achieve the above objectives, the present invention adopts the following technical solutions.

A disposable fetal scalp spiral electrode includes: an electrode fixing column, a spiral electrode, an auxiliary electrode, a twisted pair, an inner tube sleeve, an outer tube sleeve and an outer tube sleeve fixing component; a first end of the outer tube sleeve and the outer tube sleeve fixing component are in an insertion connection and the electrode fixing column is capable of extending out or retracting from a second end of the outer tube sleeve; the spiral electrode is arranged on a top end face of the electrode fixing column and the auxiliary electrode is disposed around the circumference of the electrode fixing column respectively; the inner tube sleeve is sleeved on the twisted pair and two wires of a first end of the twisted pair are respectively connected to the spiral electrode and the auxiliary electrode; a second end of the twisted pair is led out from the outer tube sleeve fixing component; the outer tube sleeve fixing component includes an inner sleeve cylinder and an outer sleeve cylinder, wherein the outer sleeve cylinder is loosely sleeved on the inner sleeve cylinder being capably of moving relatively along an axial direction; a first end of the outer sleeve cylinder and the first end of the outer tube sleeve are in an insertion connection; a fixing wire ring is operably connected to an end portion of the inner sleeve cylinder; a concave arc section is formed on the fixing wire ring and a groove corresponding to the concave arc section is formed on an edge of the end portion of the inner sleeve cylinder; a mouth configured to clamp the twisted pair is enclosed by the concave arc section and the groove when the fixing wire ring is fastened on the end portion of the inner sleeve cylinder.

An improvement to the above technical solution: the electrode fixing column is cylindrical; the auxiliary electrode is a cylindrical auxiliary electrode piece or the auxiliary electrode includes two or more arc-shaped auxiliary electrode pieces; the top end face of the electrode fixing column has a spherical shape or a flat shape with smooth edges; the spiral electrode disposed on the top end surface of the electrode fixing column is a single spiral needle.

A further improvement to the above technical solution: a positioning plate is provided at the bottom end of the electrode fixing column and edges of the positioning plate are smooth; the two wires of the twisted pair are respectively extended into the electrode fixing column along both sides of the positioning plate; the electrode fixing column, the positioning plate, the single spiral needle and the auxiliary electrode are injection-molded into an integrated structure; two clamping slots are symmetrically formed on an end portion of the inner tube sleeve close to the electrode fixing column and the positioning plate disposed at the bottom end of the electrode fixing column is inserted into the two clamping slots for positioning respectively.

A further improvement to the above technical solution: a long notch extending along the axial direction is formed on a circumferential wall of the outer sleeve cylinder and a positioning column is provided on an outer wall of the inner sleeve cylinder, wherein the positioning column extends into the long notch.

A further improvement to the above technical solution: a protruding connecting end is provided at one end of the outer sleeve cylinder and the outer diameter of the connecting end is slightly larger than the inner diameter of the outer tube sleeve; a tight fit formed between the outer tube sleeve and the connecting end of the outer sleeve cylinder.

A further improvement to the above technical solution: anti-slip ribs are provided on the outer wall of the inner sleeve cylinder and anti-slip strips extending along the axial direction is formed on the outer wall of the outer sleeve cylinder; two lugs are provided on the outer wall of one end of the outer sleeve cylinder.

A further improvement to the above technical solution: anti-slip ring-shaped strips are formed on the outer wall of the outer sleeve cylinder.

### Beneficial effect

Compared with the prior art, the present invention has the following advantages and positive effects:
1. The outer tube sleeve fixing component includes an inner sleeve cylinder and an outer sleeve cylinder, wherein the outer sleeve cylinder is loosely sleeved on the inner sleeve cylinder being capably of moving relatively along an axial direction. Further a fixing wire ring is operably connected to an end portion of the inner sleeve cylinder; a mouth configured to clamp the twisted pair is enclosed when the fixing wire ring is fastened on; therefore the operations of the electrode fixing column: extending or retracting are convenient and accurate, and the twisted pair could be fixed conveniently and reliably.
2. In the present invention, the electrode fixing column is cylindrical; the auxiliary electrode is a cylindrical auxiliary electrode piece or the auxiliary electrode includes two or more arc-shaped auxiliary electrode pieces; the top end face of the electrode fixing column has a spherical shape or a flat shape with smooth edges; the spiral electrode disposed on the top end surface of the electrode fixing column is a single spiral needle. The shape of the electrode fixing column is reasonably designed and safe to use.
3. In the present invention, a positioning plate provided at the bottom end of the electrode fixing column, the electrode fixing column, the positioning plate, the single spiral needle and the auxiliary electrode are injection-molded into an integrated structure; and the structure is compact and the manufacturing is convenient.

### Description of the drawings

Fig.1 is a perspective view of a disposable fetal scalp spiral electrode according to the present invention, in which a fixing wire ring is fastened on.
Fig.2 is an assembly schematic diagram of a first embodiment of an outer tube sleeve fixing component of a disposable fetal scalp spiral electrode according to the present invention.
Fig.3 is an assembly schematic diagram of a second embodiment of an outer tube sleeve fixing component of a disposable fetal scalp spiral electrode according to the present invention.
Fig.4 is an assembly schematic diagram of a third embodiment of an outer tube sleeve fixing component of a disposable fetal scalp spiral electrode according to the present invention.
Fig.5 is a perspective view of a disposable fetal scalp spiral electrode according to the present invention, in which the electrode fixing column extends out.
Fig.6 is a perspective view of a first embodiment of an electrode fixing column of a disposable fetal scalp spiral electrode according to the present invention.
Fig.7 is a perspective view of a second embodiment of an electrode fixing column of a disposable fetal scalp spiral electrode according to the present invention.

In the figures: 1. outer tube sleeve; 2. outer sleeve cylinder; 2.1. lug; 2.2. long notch with an opening; 2.3. anti-slip strips; 2.4. connecting end; 2.5. closed long notch; 2.6. anti-slip ring-shaped strips; 3.fixing wire ring; 3.1. concave arc section; 4. twisted pair; 5. inner sleeve cylinder; 5.1. anti-slip ribs; 5.2. positioning column; 5.3, groove; 6, electrode fixing column 6.1. arc-shaped auxiliary electrode pieces; 6.2. positioning plate; 6.3. cylindrical auxiliary electrode piece; 7. single spiral needle; 8. inner tube sleeve; 8.1. clamping slots.

### Detailed Description

To make the purpose, technical solutions and advantages of embodiments of the present invention clearer, the technical solutions in the embodiments of the present invention will be described clearly and completely in conjunction with accompanying drawings of the embodiments of the present invention.

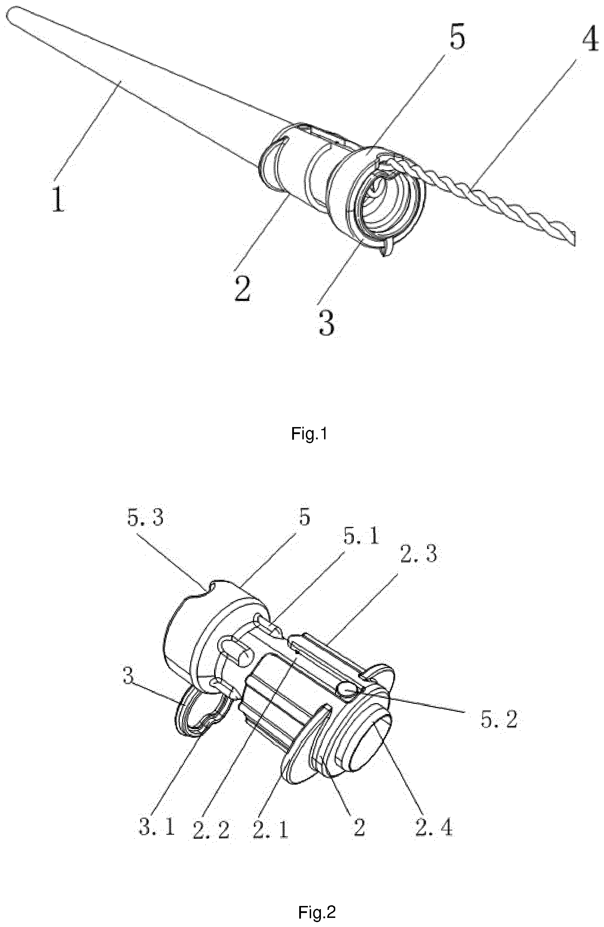

A disposable fetal scalp spiral electrode according the present invention is shown in Fig.1 to Fig.7, which includes: an electrode fixing column 6, a spiral electrode, an auxiliary electrode, a twisted pair 4, an outer tube sleeve 1 and an outer tube sleeve fixing component. A first end of the outer tube sleeve 1 and the outer tube sleeve fixing component are in an insertion connection and the electrode fixing column 6 is capable of extending out or retracting from a second end of the outer tube sleeve 1. Respectively the spiral electrode is arranged on a top end face of the electrode fixing column 6 and the auxiliary electrode is disposed around the circumference of the electrode fixing column 6. An inner tube sleeve 8 is sleeved on the twisted pair 4 and two wires of a first end of the twisted pair 4 are respectively connected to the spiral electrode and the auxiliary electrode. A second end of the twisted pair 4 is led out from the outer tube sleeve fixing component. The outer tube sleeve fixing component includes an inner sleeve cylinder 5 and an outer sleeve cylinder 2, wherein the outer sleeve cylinder 2 is loosely sleeved on the inner sleeve cylinder 5 thereby being capable of moving relatively along an axial direction. A first end of the outer sleeve cylinder 2 and the first end of the outer tube sleeve 1 are in an insertion connection; the second end of the twisted pair 4 is drawn out from the outer tube sleeve fixing component and the electrode fixing column 6 is capable of extending out or retracting from the second end of the outer tube sleeve 1. The first end of the outer sleeve cylinder 2 and the first end of the outer tube sleeve 1 are in an insertion connection and a fixing wire ring 3 is operably connected to an end portion of the inner sleeve cylinder 5. A concave arc section 3.1 is formed on the fixing wire ring 3 while a groove 5.3 corresponding to the concave arc section 3.1 is formed on an edge of the end portion of the inner sleeve cylinder 5. A mouth configured to clamp the twisted pair 4 is enclosed by the concave arc section 3.1 and the groove 5.3 when the fixing wire ring 3 is fastened on the end portion of the inner sleeve cylinder 5.

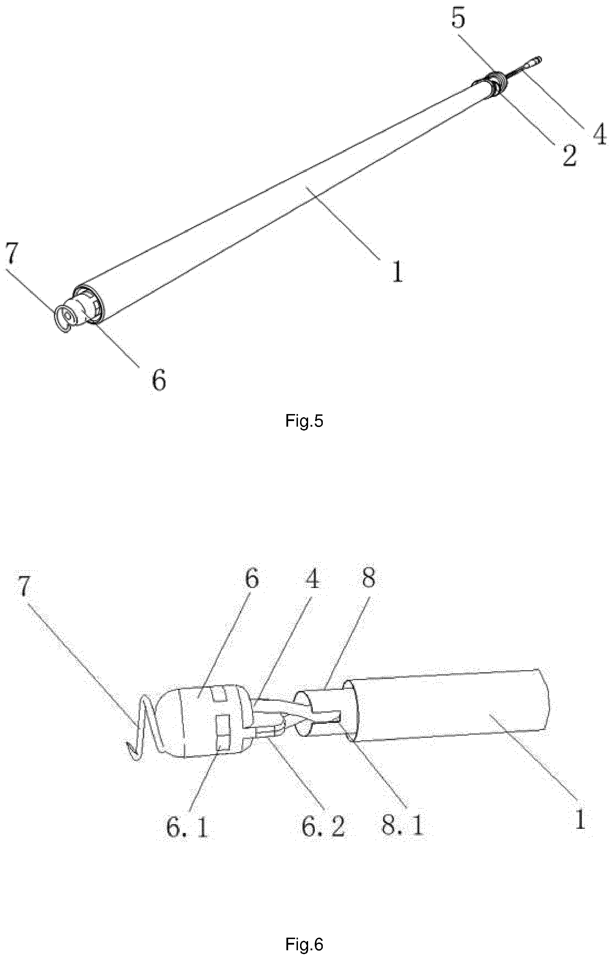

To be specific, as shown in Fig.6, the electrode fixing column 6 is cylindrical; the auxiliary electrode disposed around the circumference of the electrode fixing column includes two or more arc-shaped auxiliary electrode pieces 6.1. The top end face of the electrode fixing column 6 has a spherical shape and the spiral electrode disposed on the top of the spherical top end face is a single spiral needle 7. A positioning plate 6.2 is provided at a bottom end of the electrode fixing column 6 and the edge of the positioning plate 6.2 is smooth. The two wires of the twisted pair 4 are respectively extended into the electrode fixing column 6 along both sides of the positioning plate 6.2. Two clamping slots 8.1 are symmetrically formed on an end portion of the inner tube sleeve 8 close to the electrode fixing column 6 and the positioning plate 6.2 disposed at the bottom end of the electrode fixing column 6 is inserted into the two clamping slots 8.1 for positioning respectively.

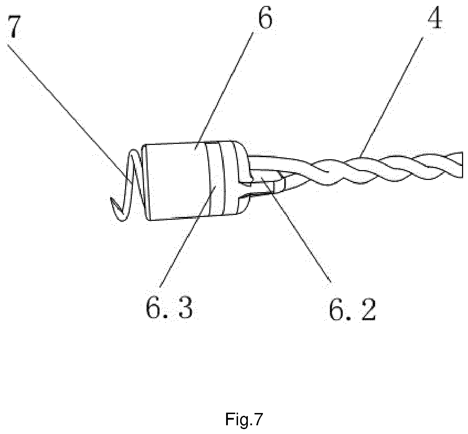

Alternatively, as shown in Fig.7, the electrode fixing column 6 is cylindrical; the auxiliary electrode disposed around the circumference of the electrode fixing column is a cylindrical auxiliary electrode piece 6.3. The top end face of the electrode fixing column 6 has a flat shape with smooth edge and the spiral electrode connected to the flat top end face is a single spiral needle 7. A positioning plate 6.2 is provided at the bottom end of the electrode fixing column 6 and the edge of the positioning plate 6.2 is smooth. The two wires of the twisted pair 4 are respectively extended into the electrode fixing column 6 along both sides of the positioning plate 6.2.

To facilitate the manufacturing, the electrode fixing column 6, the positioning plate 6.2, the single spiral needle 7 and the arc-shaped auxiliary electrode pieces 6.1 or the cylindrical auxiliary electrode piece 6.3 are injection-molded into an integrated structure; the electrode fixing column 6 and the positioning plate 6.2 are made of plastic.

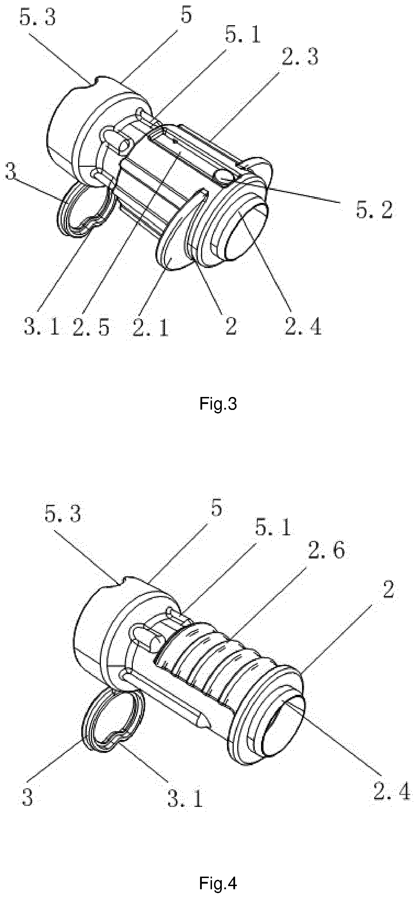

A long notch extending along the axial direction is formed on a circumferential wall of the outer sleeve cylinder 2 and a positioning column 5.2 is provided on an outer wall of the inner sleeve cylinder 5, wherein the positioning column 5.2 extends into the long notch to maintain the outer sleeve cylinder 2 and the inner sleeve cylinder 5 only could move relative to each other in the axial direction within a limited length. As shown in Fig.2, the long notch extending along the axial direction formed on the circumferential wall of the outer sleeve cylinder 2 could be a long notch 2.2 with an opening; or as shown in Fig.3, the long notch extending along the axial direction formed on the circumferential wall of the outer sleeve cylinder 2 could be a closed long notch 2.5.

With reference to Fig.2 to Fig.4, a protruding connecting end 2.4 is provided at one end of the outer sleeve cylinder 2, and the outer diameter of the connecting end 2.4 is slightly larger than the inner diameter of the outer tube sleeve 1; a tight fit formed between the outer tube sleeve 1 and the connecting end 2.4 of the outer sleeve cylinder 2.

In order to avoid slippery in use, as shown in Fig.2 and Fig.3, anti-slip ribs 5.1 are provided on the outer wall of the inner sleeve cylinder 5 and anti-slip strips 2.3 extending along the axial direction is formed on the outer wall of the outer sleeve cylinder 2. Two lugs 2.1 are provided on the outer wall of one end of the outer sleeve cylinder 2 in order to push or pull the outer sleeve cylinder 2 by two fingers.

In order to avoid slippery in use, as shown in Fig.4, anti-slip ring-shaped strips 2.6 are formed on the outer wall of the outer sleeve cylinder 2. With this arrangements, the outer sleeve cylinder 2 could be pushed or pulled easily even without the lugs and the overall structure is more concise.

When the disposable fetal scalp spiral electrode is being used, the outer sleeve cylinder 2 could be moved relative to the inner sleeve cylinder 5 for a certain distance by hand to expose the electrode fixing column 6 from the second end of the outer tube sleeve 1. The twisted pair 4 drawn from the first end of the outer tube sleeve 1 is inserted into the groove 5.3, and the fixing wire ring 3 is buckled on the end portion of the inner sleeve cylinder 5 to clamp the twisted pair 4 into the mouth formed by the concave arc section 3.1 and the groove 5.3. When the disposable fetal scalp spiral electrode is not being used, the fixing wire ring 3 is disconnected from the end portion of the inner sleeve cylinder 5, the outer sleeve cylinder 2 could be moved relative to the inner sleeve cylinder 5 for a certain distance along an opposite direction by hand to hide the electrode fixing column 6 in the second end of the outer tube sleeve 1. The twisted pair 4 drawn from the first end of the outer tube sleeve 1 is inserted into the groove 5.3, and the fixing wire ring 3 is buckled on the end portion of the inner sleeve cylinder 5 to clamp the twisted pair 4 into the mouth formed by the concave arc section 3.1 and the groove 5.3.

The above are only the preferred embodiments of the present invention, and are not intended to limit the present invention presented in other forms. Any person familiar with the technical field may use the content disclosed above to change or modify it into equivalent embodiments. Any simple modifications, equivalent changes and modifications made to the above embodiments based on the essence of the present invention without departing from the content of the technical solution of the present invention still belong to the protection scope of the technical solution of the present invention.

## Claims

1. A disposable fetal scalp spiral electrode includes: an electrode fixing column, a spiral electrode, an auxiliary electrode, a twisted pair, an inner tube sleeve, an outer tube sleeve and an outer tube sleeve fixing component; a first end of the outer tube sleeve and the outer tube sleeve fixing component are in an insertion connection and the electrode fixing column is capable of extending out or retracting from a second end of the outer tube sleeve; the spiral electrode is arranged on a top end face of the electrode fixing column and the auxiliary electrode is disposed around the circumference of the electrode fixing column respectively; the inner tube sleeve is sleeved on the twisted pair and two wires of a first end of the twisted pair are respectively connected to the spiral electrode and the auxiliary electrode; a second end of the twisted pair is led out from the outer tube sleeve fixing component;
**characterized in that**: the outer tube sleeve fixing component includes an inner sleeve cylinder and an outer sleeve cylinder, wherein the outer sleeve cylinder is loosely sleeved on the inner sleeve cylinder being capably of moving relatively along an axial direction; a first end of the outer sleeve cylinder and the first end of the outer tube sleeve are in an insertion connection; a fixing wire ring is operably connected to an end portion of the inner sleeve cylinder; a concave arc section is formed on the fixing wire ring and a groove corresponding to the concave arc section is formed on an edge of the end portion of the inner sleeve cylinder; a mouth configured to clamp the twisted pair is enclosed by the concave arc section and the groove when the fixing wire ring is fastened on the end portion of the inner sleeve cylinder.

2. A disposable fetal scalp spiral electrode according to claim 1, **characterized in that**: the electrode fixing column is cylindrical; the auxiliary electrode is a cylindrical auxiliary electrode piece or the auxiliary electrode includes two or more arc-shaped auxiliary electrode pieces; the top end face of the electrode fixing column has a spherical shape or a flat shape with smooth edges; the spiral electrode disposed on the top end surface of the electrode fixing column is a single spiral needle.

3. A disposable fetal scalp spiral electrode according to claim 2, **characterized in that**: a positioning plate is provided at the bottom end of the electrode fixing column and edges of the positioning plate are smooth; the two wires of the twisted pair are respectively extended into the electrode fixing column along both sides of the positioning plate; the electrode fixing column, the positioning plate, the single spiral needle and the auxiliary electrode are injection-molded into an integrated structure; two clamping slots are symmetrically formed on an end portion of the inner tube sleeve close to the electrode fixing column and the positioning plate disposed at the bottom end of the electrode fixing column is inserted into the two clamping slots for positioning respectively.

4. A disposable fetal scalp spiral electrode according to any one of claim 1 to 3, **characterized in that**, a long notch extending along the axial direction is formed on a circumferential wall of the outer sleeve cylinder and a positioning column is provided on an outer wall of the inner sleeve cylinder, wherein the positioning column extends into the long notch.

5. A disposable fetal scalp spiral electrode according to any one of claim 1 to 3, **characterized in that**, a protruding connecting end is provided at one end of the outer sleeve cylinder and the outer diameter of the connecting end is slightly larger than the inner diameter of the outer tube sleeve; a tight fit formed between the outer tube sleeve and the connecting end of the outer sleeve cylinder.

6. A disposable fetal scalp spiral electrode according to claim 4, **characterized in that**, a long notch extending along the axial direction is formed on a circumferential wall of the outer sleeve cylinder and a positioning column is provided on an outer wall of the inner sleeve cylinder, wherein the positioning column extends into the long notch.

7. A disposable fetal scalp spiral electrode according to any one of claim 1 to 3, **characterized in that**, anti-slip ribs are provided on the outer wall of the inner sleeve cylinder and anti-slip strips extending along the axial direction is formed on the outer wall of the outer sleeve cylinder; two lugs are provided on the outer wall of one end of the outer sleeve cylinder.

8. A disposable fetal scalp spiral electrode according to claim 4, **characterized in that**, anti-slip ribs are provided on the outer wall of the inner sleeve cylinder and anti-slip strips extending along the axial direction is formed on the outer wall of the outer sleeve cylinder; two lugs are provided on the outer wall of one end of the outer sleeve cylinder.

9. A disposable fetal scalp spiral electrode according to any one of claim 1 to 3, **characterized in that**, anti-slip ring-shaped strips are formed on the outer wall of the outer sleeve cylinder.

10. A disposable fetal scalp spiral electrode according to claim 6, **characterized in that**, anti-slip ring-shaped strips are formed on the outer wall of the outer sleeve cylinder.
